# EUROPÄISCHE PATENTSCHRIFT

(11) **EP 1 347 977 B1**
(45) Veröffentlichungstag und Bekanntmachung des Hinweises auf die Patenterteilung: **01.09.2004**
(21) Anmeldenummer: 01271373.1
(22) Anmeldetag: 05.12.2001
(51) Int. Cl.: C07D 487/04, A61K 31/53, A61P 15/00

(54) **VERFAHREN ZUR HERSTELLUNG VON 2-(2-ETHOXYPHENYL)-SUBSTITUIERTEN IMIDAZOTRIAZINONEN**
METHOD FOR THE PRODUCTION OF 2-(2-ETHOXYPHENYL)-SUBSTITUTED IMIDAZOTRIAZINONES
PROCEDE DE PRODUCTION D'IMIDAZOTRIAZINONES 2-(2-ETHOXYPHENYL)-SUBSTITUES

(30) Priorität: 18.12.2000 DE 10063106
(43) Veröffentlichungstag der Anmeldung: 01.10.2003
(73) Patentinhaber: Bayer HealthCare AG, 51368 Leverkusen (DE)
(72) Erfinder: NOWAKOWSKI, Marc, 42115 Wuppertal (DE); VETTER, Alexander, 51061 Köln (DE)
(86) Internationale Anmeldenummer: PCT/EP2001/014231
(87) Internationale Veröffentlichungsnummer: WO 2002/050075

(56) Entgegenhaltungen:
- WO-A-99/24433
- US-A- 4 278 673
- CHARLES I ET AL: "BICYCLIC HETEROCYCLES WITH NITROGEN AT THE RING JUNCTION. PART 2.1 APPLICATION OF THE DAKIN-WEST REACTION TO THE SYNTHESIS OF IMIDAZO-U5,1-F-1,2,4-TRIAZIN-4(3H)-ONES" JOURNAL OF THE CHEMICAL SOCIETY, PERKIN TRANSACTIONS 1, CHEMICAL SOCIETY. LETCHWORTH, GB, Nr. 5, 1. Mai 1980 (1980-05-01), Seiten 1139-1146, XP002027191 ISSN: 1472-7781
- COCCO ET AL.: "1-Acylaminoimidazoles Synthesis and Antimicrobial Activity" IL PHARMACO, Bd. 47, Nr. 2, 1992, Seiten 229-238, XP002191792

## Beschreibung

Die vorliegende Erfindung betrifft ein Verfahren zur Herstellung von 2-(2-Ethoxyphenyl)-substituierten Imidazotriazinonen.

Es ist bekannt, dass Verbindungen. welche in der Lage sind, cyclisches Guanosin-3',5'-monophosphat metabolisierende Phosphodiesterasen (cGMP-PDE's) zu inhibieren, zur Behandlung von Impotenz eingesetzt werden können (vgl. z.B. EP-B-0 702 555; K. Murray, Drugs, News & Perspectives 6 (1993), 150).

In der WO 99/24433 sind Sulfonamid-substituierte Imidazotriazinone als potente Inhibitoren von entweder einer oder mehrerer der cyclisches Guanosin-3',5'-monophosphat metabolisierenden Phosphodiesterasen (cGMP-PDE's) beschrieben. Entsprechend der Nomenklatur von Beavo und Reifsnyder (Trends in Pharmacol. Sci. 11, 150-155, 1990) handelt es sich bei diesen cGMP-PDE's um die Phosphodiesterase-Isoenzyme PDE-I, PDE-II und PDE-V.

Die in der WO 99/24433 beschriebenen Sulfonamid-substituierten Imidazotriazinone werden aus entsprechenden 2-Ethoxyphenyl-substituierten Imidazotriazinonen durch Umsetzung mit Chlorsulfonsäure und anschließende Reaktion mit einem entsprechenden Amin hergestellt. Die hierfür als Zwischenstufen benötigten 2-Ethoxyphenyl-substituierten Imidazotriazinone werden gemäß der WO 99/24433 durch Umsetzung einer Verbindung der Formel (1) 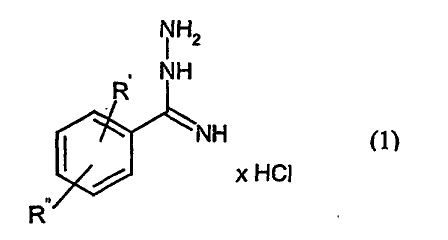 worin
- R' und R": gleich oder verschieden sind und für Wasserstoff, geradkettiges oder verzweigtes Alkyl mit bis zu 6 Kohlenstoffatomen, Hydroxy oder für geradkettiges oder verzweigtes Alkoxy mit bis zu 6 Kohlenstoffatomen stehen,
mit einer Verbindung der Formel (2) 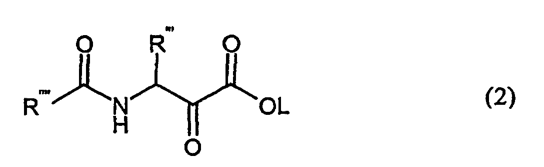 in welcher
- R''': für Wasserstoff oder geradkettiges oder verzweigtes Alkyl mit bis zu 4 Kohlenstoffatomen steht,
- R'''': für geradkettiges Alkyl mit bis zu 4 Kohlenstoffatomen steht,
- L: für geradkettiges oder verzweigtes Alkyl mit bis zu 4 Kohlenstoffatomen steht,
hergestellt. Die Verbindungen der Formel (1) werden hierbei über eine dreistufige Synthese aus den entsprechenden Benzonitrilen gewonnen. Die Verbindungen der Formel (2) werden in zwei Stufen aus den entsprechenden Alkylcarbonsäurehalogeniden und α-Aminosäuren hergestellt.

Bei diesem Verfahren müssen reaktive Zwischenstufen durchlaufen werden, wodurch sich die Umsetzung dieser Synthese im großtechnischen Maßstab gegebenenfalls als schwierig erweisen kann.

Es war daher die Aufgabe der vorliegenden Erfindung, ein Verfahren zur Herstellung von 2-(2-Ethoxyphenyl)-substituierten Imidazotriazinonen bereitzustellen, welches durch Vermeidung reaktiver Zwischenstufen einen einfachen Produktionsablauf im technischen Maßstab ermöglicht.

Diese Aufgabe wird gemäß der vorliegenden Erfindung durch ein Verfahren gemäß Anspruch 1 gelöst.

Im einzelnen umfasst das erfindungsgemäße Verfahren zur Herstellung von Verbindungen der Formel (I) 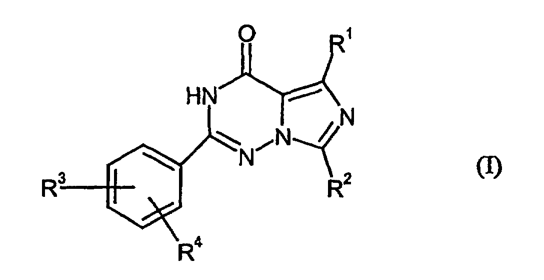 worin
- R¹: für Wasserstoff oder geradkettiges oder verzweigtes Alkyl mit bis zu 4 Kohlenstoffatomen steht,
- R²: für geradkettiges Alkyl mit bis zu 4 Kohlenstoffatomen steht,
- R³ und R⁴: gleich oder verschieden sind und für Wasserstoff, geradkettiges oder verzweigtes Alkyl mit bis zu 6 Kohlenstoffatomen, Hydroxy oder für geradkettiges oder verzweigtes Alkoxy mit bis zu 6 Kohlenstoffatomen stehen,
die Umsetzung von Verbindungen der Formel (II) 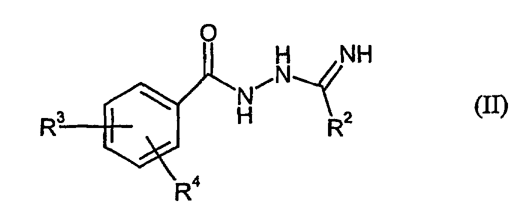 worin R², R³ und R⁴ die vorstehend angegebene Bedeutung haben,
mit Verbindungen der Formel (III) 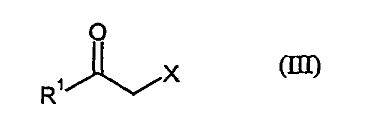 worin
- R¹: die vorstehend angegebene Bedeutung hat,
- X: für Halogen steht;
in Gegenwart einer Base und gegebenenfalls eines Metalliodids in einem organischen Lösungsmittel zu Verbindungen der Formel (IV) 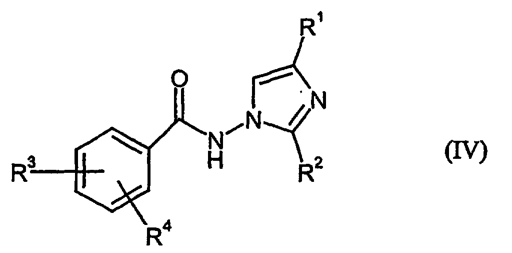 worin R¹, R², R³ und R⁴ die vorstehend angegebene Bedeutung haben,
und die anschließende Umsetzung
[A] mit Iod in Gegenwart einer Base in einem Lösungsmittel, anschließende Reaktion mit einem Metallcyanid in einem Lösungsmittel und Umsetzung mit einer Säure
   oder
[B] mit Brom im sauren Medium, anschließende Reaktion mit einem Metallcyanid in einem Lösungsmittel und Umsetzung mit einer Säure.

Gemäß einer bevorzugten Ausführungsform der vorliegenden Erfindung bedeuten bei den Reaktanden und dem Endprodukt des erfindungsgemäßen Verfahrens
- R¹: geradkettiges Alkyl mit bis zu 4 Kohlenstoffatomen,
- R²: für geradkettiges Alkyl mit bis zu 4 Kohlenstoffatomen,
- R³ und R⁴: gleich oder verschieden voneinander Wasserstoff oder geradkettiges oder verzweigtes Alkoxy mit bis zu 4 Kohlenstoffatomen.

Gemäß einer besonders bevorzugten Ausführungsform der vorliegenden Erfindung bedeuten bei den Reaktanden und dem Endprodukt des erfindungsgemäßen Verfahrens
- R¹: Methyl oder Ethyl,
- R²: n-Propyl,
- R³: Wasserstoff
- R⁴: Ethoxy.

Im Rahmen der vorliegenden Erfindung haben die Substituenten soweit nicht anders angegeben im allgemeinen die folgende Bedeutung:
Alkyl steht im allgemeinen für einen geradkettigen oder verzweigten Kohlenwasserstoffrest mit 1 bis 6 Kohlenstoffatomen. Beispielsweise seien Methyl, Ethyl, Propyl, Isopropyl, Butyl, Isobutyl, Pentyl, Isopentyl, Hexyl, Isohexyl genannt.
Alkoxy steht im allgemeinen für einen über ein Sauerstoffatom gebundenen geradkettigen oder verzweigten Kohlenwasserstoffrest mit 1 bis 6 Kohlenstoffatomen. Beispielsweise seien Methoxy, Ethoxy, Propoxy, Isopropoxy, Butoxy, Isobutoxy, Pentoxy Isopentoxy, Hexoxy, Isohexoxy genannt. Die Begriffe "Alkoxy" und "Alkyloxy" werden synonym verwendet.
Halogen steht im Rahmen der Erfindung für Fluor, Chlor, Brom und Iod.

Die Verbindungen der Formel (II) können gemäß der vorliegenden Erfindung hergestellt werden durch Umsetzung von Verbindungen der Formel (V) 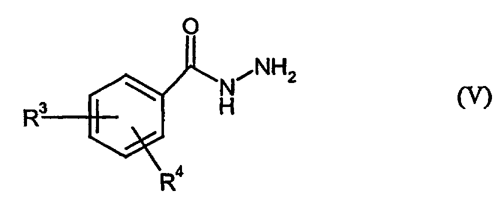 worin
- R³ und R⁴: die vorstehend angegebenen Bedeutungen haben,
mit Verbindungen der Formel (VI) 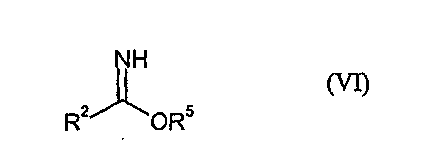 worin
- R²: die vorstehend angegebene Bedeutung hat
- R⁵: für C₁₋₆-Alkyl steht,
in einem organischen Lösungsmittel in Gegenwart einer Base.

Die Verbindungen der Formel (V) können aus den entsprechenden Benzoesäureestern durch Umsetzung mit Hydrazinhydrat nach dem Fachmann bekannten Methoden hergestellt werden (vgl. J. March, Advanced Organic Chemistry, 3^{rd} ed., Wiley, 1985, S. 375). Die Benzoesäureester sind bekannt oder nach dem Fachmann geläufigen Methoden herstellbar.

Die Verbindungen der Formel (VI) können aus den entsprechenden käuflichen Alkylnitrilen auf bekannte Weise durch Umsetzung mit HCl und einem Alkohol hergestellt werden (Pinner-Reaktion).

Die Umsetzung der Verbindungen der Formel (V) mit Verbindungen der Formel (VI) zu den Verbindungen der Formel (II) wird in einem organischen Lösungsmittel in Gegenwart einer Base, beispielsweise einer organischen Base wie einem Amin, vorzugsweise Triethylamin, vorzugsweise bei Normaldruck und - nach Zusammengeben der Reaktanden unter Abkühlung auf beispielsweise -20°C bis +5°C, vorzugsweise 0°C - Rühren der Reaktionslösung für mehrere Stunden, beispielsweise 2 bis 60 Stunden, vorzugsweise 24 bis 50 Stunden, bei Raumtemperatur und anschließende Überfühung in das Hydrochlorid zur Isolierung durch Zutropfen von Salzsäure unter Abkühlung auf beispielsweise -20°C bis +5°C, vorzugsweise 0°C durchgeführt. Die Reaktanden können hierbei je nach ihrer Beschaffenheit in äquimolaren Mengen eingesetzt werden, oder einer der Reaktanden wird in bis zu dreifachem Überschuss eingesetzt.

Als Lösungsmittel für diese Reaktion eignen sich die üblichen organischen Lösungsmittel, die sich unter den Reaktionsbedingungen nicht verändern. Hierzu gehören bevorzugt Alkohole wie Methanol, Ethanol oder iso-Propanol, Ether wie Diethylether, Dioxan, Tetrahydrofuran, Glykoldimethylether, oder Kohlenwasserstoffe wie Benzol, Toluol, Xylol, Hexan, Cyclohexan oder Erdölfrakionen, oder Halogenkohlenwasserstoffe wie Dichlormethan, Trichlormethan, Tetrachlormethan, Dichlorethan, Trichlorethylen oder Chlorbenzol, oder Essigester, Dimethylformamid, Hexamethylphosphorsäuretriamid, Acetonitril, Aceton, Dimethoxyethan oder Pyridin. Ebenso ist es möglich, Gemische der genannten Lösemittel zu verwenden. Besonders bevorzugt ist iso-Propanol.

Die Reaktion wird im allgemeinen bei Normaldruck durchgeführt. Es ist aber auch möglich, bei Überdruck oder bei Unterdruck zu arbeiten (z.B. in einem Bereich von 0,5 bis 5 bar).

Die Umsetzung der Verbindungen der Formel (II) mit Verbindungen der Formel (III) zu den Verbindungen der Formel (IV) kann erfindungsgemäß auf zwei Wegen erfolgen.

Einerseits kann die Reaktion dergestalt durchgeführt werden, dass die Verbindungen der Formel (II) in Form der entsprechenden freien Base (aus dem Hydrochlorid der Verbindungen der Formel (II) durch Reaktion mit einer Base wie einer Alkali- oder Erdalkalimetallbase, vorzugsweise einem Alkali- oder Erdalkalimetallcarbonat wie Natriumhydrogencarbonat erhältlich) mit Verbindungen der Formel (III) in einem organischen Lösungsmittel in Gegenwart einer Base und gegebenenfalls eines Metalliodids vorzugsweise bei Normaldruck und Rühren der Reaktionslösung für mehrere Stunden, beispielsweise 2 bis 12 Stunden, vorzugsweise 3 bis 6 Stunden, bei erhöhter Temperatur, beispielsweise 30 bis 80°C, vorzugsweise 40 bis 60°C, insbesondere 50°C, zu den Verbindungen der Formel (IV) umgesetzt werden. Die Reaktanden können hierbei je nach ihrer Beschaffenheit in äquimolaren Mengen eingesetzt werden, oder die Verbindung der Formel (III) wird in bis zu dreifachem Überschuss eingesetzt.

Die Verbindungen der Formel (III) sind entweder käuflich erhältlich oder auf dem Fachmann bekannte Weise durch eine α-Halogenierungsreaktion der entsprechenden käuflichen Aldehyde oder Ketone zugänglich (vgl. J. March, Advanced Organic Chemistry, 3^{rd} ed., Wiley, 1985, S. 529 f.f.). Bevorzugt werden bei beiden Varianten (der Amin- und der Carbonatvariante) die entsprechenden Chloraldehyde oder -ketone der Formel (III) eingesetzt.

Als Lösungsmittel für diese Reaktion eignen sich die üblichen organischen Lösungsmittel, die sich unter den Reaktionsbedingungen nicht verändern. Hierzu gehören bevorzugt Alkohole wie Methanol, Ethanol oder iso-Propanol, Ether wie Diethylether, Dioxan, Tetrahydrofuran, Glykoldimethylether, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethan, Trichlorethylen oder Chlorbenzol, oder Essigester, Dimethylformamid, Hexamethylphosphorsäuretriamid, Acetonitril, Aceton, Dimethoxyethan oder Pyridin. Ebenso ist es möglich, Gemische der genannten Lösemittel zu verwenden. Besonders bevorzugt ist Acetonitril.

Die Reaktion wird im allgemeinen bei Normaldruck durchgeführt. Es ist aber auch möglich, bei Überdruck oder bei Unterdruck zu arbeiten (z.B. in einem Bereich von 0,5 bis 5 bar).

Als Basen eignen sich insbesondere cyclische Amine, wie beispielsweise Piperidin, Pyridin, Dimethylaminopyridin oder C₁-C₄-Alkylamine, wie beispielsweise Triethylamin. Bevorzugt ist Triethylamin. Die Base wird im allgemeinen in einer Menge von 1 mol bis 4 mol, bevorzugt in äquimolarer Menge jeweils bezogen auf 1 mol der Verbindung der Formel (II) eingesetzt.

Als Metalliodid können alle ionischen Iodide eingesetzt werden. Erfindungsgemäß bevorzugt sind Alkali- oder Erdalkalimetalliodide wie insbesondere Kaliumiodid.

Es ist aber auch möglich, in zwei Teilschritten die Verbindungen der Formel (II) in Form des entsprechenden Hydrochlorids mit Verbindungen der Formel (III) zunächst in einem organischen Lösungsmittel in Gegenwart einer Base vorzugsweise bei Normaldruck und Rühren der Reaktionslösung für beispielsweise 1 bis 30 Stunden, vorzugsweise 12 bis 24 Stunden, bei 0 bis 25°C, vorzugsweise 10 bis 20°C, insbesondere 15°C, umzusetzen, und die erhaltenen Zwischenstufen dann in einem weiteren inerten organischen Lösungsmittel bei erhöhter Temperatur, beispielsweise 50 bis 200°C, vorzugsweise 70 bis 150°C, insbesondere 85 bis115°C, zu den Verbindungen der Formel (IV) umzusetzen. Die Reaktanden können hierbei je nach ihrer Beschaffenheit in äquimolaren Mengen eingesetzt werden, oder die Verbindung der Formel (III) wird in bis zu dreifachem Überschuss eingesetzt.

Als Lösungsmittel für diese Reaktion eignen sich die üblichen organischen Lösungsmittel, die sich unter den Reaktionsbedingungen nicht verändern. Hierzu gehören bevorzugt Alkohole wie Methanol, Ethanol oder iso-Propanol, Ether wie Diethylether, Dioxan, Tetrahydrofuran, Glykoldimethylether, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethan, Trichlorethylen oder Chlorbenzol, oder Essigester, Dimethylformamid, Hexamethylphosphorsäuretriamid, Acetonitril, Aceton, Dimethoxyethan oder Pyridin. Ebenso ist es möglich, Gemische der genannten Lösemittel zu verwenden. Besonders bevorzugt ist Aceton für den ersten Teilschritt und Xylol für den zweiten Teilschritt.

Die Reaktion wird im allgemeinen bei Normaldruck durchgeführt. Es ist aber auch möglich, bei Überdruck oder bei Unterdruck zu arbeiten (z.B. in einem Bereich von 0,5 bis 5 bar).

Als Basen eignen sich im allgemeinen Alkali- oder Erdalkalimetallcarbonate wie insbesondere Kaliumcarbonat. Die Base wird im allgemeinen in einer Menge von 1 mol bis 4 mol, bevorzugt in einer Menge von 2 mol jeweils bezogen auf 1 mol der Verbindung der Formel (II) eingesetzt.

Die so erhaltenen Verbindungen der Formel (IV) können erfindungsgemäß auf zwei Wegen in die erfindungsgemäßen Verbindungen der Formel (I) überführt werden, wobei sich diese Wege nur im ersten Schritt voneinander unterscheiden.

Der erste Schritt besteht bei beiden Wegen in einer Halogenierung der 5-Position des Imidazolrings. Im ersten Weg erfolgt diese Halogenierung durch Umsetzung der Verbindungen der Formel (IV) mit Iod in Gegenwart einer Base in einem Lösungsmittel vorzugsweise bei Normaldruck und Rühren der Reaktionslösung für beispielsweise 1 bis 48 Stunden, vorzugsweise 12 bis 36 Stunden, insbesondere 24 Stunden bei Raumtemperatur unter Lichtausschluss. Das Iod wird hierbei vorzugsweise im Überschuss, beispielsweise einem zwei- bis vierfachen Überschuss eingesetzt.

Als Lösungsmittel für diese Reaktion eignen sich die für Halogenierungsreaktionen üblichen Lösungsmittel, die sich unter den Reaktionsbedingungen nicht verändern. Hierzu gehören bevorzugt Alkohole wie Methanol, Ethanol oder iso-Propanol, Ether wie Diethylether, Dioxan, Tetrahydrofuran, Glykoldimethylether, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethan, Trichlorethylen oder Chlorbenzol, oder Essigester, Dimethylformamid, Hexamethylphosphorsäuretriamid, Acetonitril, Aceton, Dimethoxyethan oder Pyridin oder Wasser. Ebenso ist es möglich, Gemische der genannten Lösemittel zu verwenden. Besonders bevorzugt ist erfindungsgemäß ein Dioxan/Wasser-Gemisch.

Die Reaktion wird im allgemeinen bei Normaldruck durchgeführt. Es ist aber auch möglich, bei Überdruck oder bei Unterdruck zu arbeiten (z.B. in einem Bereich von 0,5 bis 5 bar).

Als Basen eignen sich im allgemeinen Alkali- oder Erdalkalimetallcarbonate wie insbesondere Natriumcarbonat. Die Base wird im allgemeinen in einer Menge von 1 mol bis 10 mol, bevorzugt in einer Menge von 2 mol bis 6 mol jeweils bezogen auf 1 mol der Verbindung der Formel (IV) eingesetzt.

Im zweiten Weg erfolgt diese Halogenierung durch Umsetzung der Verbindungen der Formel (IV) mit Brom im sauren Medium vorzugsweise bei Normaldruck und Rühren der Reaktionslösung für beispielsweise 1 bis 24 Stunden, vorzugsweise 1 bis 12 Stunden, insbesondere 1 bis 6 Stunden bei Raumtemperatur. Das Brom wird hierbei vorzugsweise im Überschuss, beispielsweise einem bis zu zweifachen Überschuss eingesetzt.

Die Reaktion wird im allgemeinen bei Normaldruck durchgeführt. Es ist aber auch möglich, bei Überdruck oder bei Unterdruck zu arbeiten (z.B. in einem Bereich von 0,5 bis 5 bar).

Als Säuren können schwache organische Carbonsäuren wie beispielsweise Alkancarbonsäuren, insbesondere Essigsäure eingesetzt werden, welche mit den Verbindungen der Formel (IV) keine unerwünschten Nebenreaktionen eingehen.

Anschließend werden die so erhaltenen Iod- oder Brom-Verbindungen mit einem Metallcyanid in einem Lösungsmittel vorzugsweise bei Normaldruck und Rühren der Reaktionslösung für mehrere Stunden, beispielsweise 2 bis 12 Stunden, vorzugsweise 3 bis 6 Stunden, bei erhöhter Temperatur, beispielsweise 30 bis 120°C, vorzugsweise 60 bis 110°C, insbesondere 100°C, zu den entsprechenden Nitrilen umgesetzt werden.

Als Lösungsmittel für diese Reaktion eignen sich die für derartige Reaktionen üblichen organischen Lösungsmittel, die sich unter den Reaktionsbedingungen nicht verändern. Hierzu gehören bevorzugt Alkohole wie Methanol, Ethanol oder iso-Propanol, Ether wie Diethylether, Dioxan, Tetrahydrofuran, Glykoldimethylether, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethan, Trichlorethylen oder Chlorbenzol, oder Essigester, Dimethylformamid, Hexamethylphosphorsäuretriamid, Acetonitril, Aceton, Dimethoxyethan oder Pyridin oder Wasser. Ebenso ist es möglich, Gemische der genannten Lösemittel zu verwenden. Besonders bevorzugt ist Pyridin.

Die Reaktion wird im allgemeinen bei Normaldruck durchgeführt. Es ist aber auch möglich, bei Überdruck oder bei Unterdruck zu arbeiten (z.B. in einem Bereich von 0,5 bis 5 bar).

Als Metallcyanide können die üblichen bei der Einführung einer Nitrilfunktion herangezogenen Metallcyanide verwendet werden. Erfindungsgemäß bevorzugt ist die Verwendung von CuCN. Das Metallcyanid wird im allgemeinen in einer Menge von 2 mol bis 10 mol, bevorzugt in einer Menge von 2 mol bis 8 mol jeweils bezogen auf 1 mol der entsprechenden Iod- oder Brom-Verbindung eingesetzt.

Im Fall der Verwendung der entsprechenden Brom-Verbindung ist es je nach Beschaffenheit dieser Verbindung angebracht, zusätzlich ein Metalliodid zuzugeben. Als Metalliodid können alle ionischen Iodide eingesetzt werden. Erfindungsgemäß bevorzugt sind Alkali- oder Erdallcalimetalliodide wie insbesondere Kaliumiodid. Das Metalliodid wird vorzugsweise im Unterschuss, beispielsweise in katalytischen Mengen wie einem zweifachen Unterschuss jeweils bezogen auf 1 mol der eingesetzten Brom-Verbindung zugegeben.

Aus den so erhaltenen Nitrilverbindungen können die erfindungsgemäßen Verbindungen der Formel (I) im letzten Schritt durch eine partiellen Hydrolyse der Nitrilfunktion zur entsprechenden Amidfunktion und anschließender intramolekularer Ringbildung erhalten werden. Diese Reaktion kann nach dem Fachmann bekannten Methoden durchgeführt werden (vgl. J. March, Advanced Organic Chemistry, 3^{rd} ed., Wiley, 1985, S. 788.). Erfindungsgemäß bevorzugt ist die Reaktion mit einer Mineralsäure wie Schwefelsäure unter Kühlung auf beispielsweise -20°C bis +5°C, vorzugsweise 0°C und Rühren der Reaktionslösung für mehrere Stunden, beispielsweise 2 bis 12 Stunden, vorzugsweise 3 bis 6 Stunden, zunächst bei Raumtemperatur und anschließend bei erhöhter Temperatur, beispielsweise 30 bis 120°C, vorzugsweise 60 bis 110°C, insbesondere 70°C.

Die Reaktion wird im allgemeinen bei Normaldruck durchgeführt. Es ist aber auch möglich, bei Überdruck oder bei Unterdruck zu arbeiten (z.B. in einem Bereich von 0,5 bis 5 bar).

Die erfindungsgemäßen Verbindungen sind Zwischenstufen für die Synthese bestimmter Inhibitoren cGMP metabolisierender PDE's, die in der WO 99/24433 beschrieben sind. Die Herstellung dieser cGMP PDE-Inhibitoren aus den erfindungsgemäßen Verbindungen der Formel (I) kann beispielsweise wie in der WO 99/24433 beschrieben erfolgen.

Die vorliegende Erfindung wird nachstehend anhand von nicht einschränkenden bevorzugten Beispielen und Vergleichsbeispielen näher dargestellt. Soweit nicht anderweitig angegeben, beziehen sich alle Mengenangaben auf Gewichtsprozente.

### Beispiele

^{**1**}**H-NMR-Spektren** wurden mit dem Spektrometer WP-200 SY der Firma Bruker bei Raumtemperatur gemessen. Als Lösungsmittel dienten deuteriertes Dimethylsulfoxid oder Deuterochloroform mit Tetramethylsilan als internem Standard (falls nicht anders vermerkt).

**MS-Spektren** wurden mit den Spektrometem M 40 der Firma AMD und API 150 der Firma PE/SCIEX gemessen. Angegeben wird die relative Signalintensität (in Prozent bezogen auf den Basispeak).

**HPLC-Analytik** wurde mit dem Gerät HP 1050 der Firma Hewlett Packard mit einer Säule des Typs Prodigy ODS III der Firma Phenomenex aufgenommen. Für die Ausgangsverbindungen (Bsp. A, C und D) wurde als Eluent ein Gemisch aus Acetonitril und 10 mM neutralem Phosphatpuffer (pH 7.2) verwendet. Für die übrigen Verbindungen wurde ein Eluentengemisch aus Methanol und 10 mM saurem Phosphatpuffer (pH 2.4) benutzt.

### Ausgangsverbindungen

### Beispiel A: Herstellung von 2-Ethoxybenzoesäurehydrazid

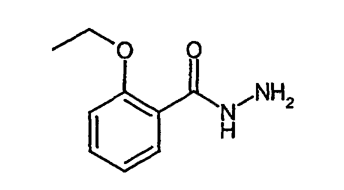

2-Ethoxybenzoesäureethylester (100 g, 128,7 mmol) und Hydrazinhydrat (100 %, 25 ml, 128,7 mmol) werden in 200 ml Ethanol gelöst und 5 Stunden unter Rückfluss erhitzt. Es werden weitere 12,5 ml (64,3 mmol) Hydrazinhydrat zugegeben und die Reaktionsmischung weitere 7 Stunden unter Rückfluss erhitzt. Zur Aufarbeitung wird die Reaktionsmischung weitgehend eingeengt und anschließend mit 300 ml Cyclohexan bei Raumtemperatur verrührt. Nach dem Abkühlen auf 0°C werden die Kristalle über eine Nutsche abgesaugt, zweimal mit je 50 ml kaltem Cyclohexan gewaschen und abschließend über Nacht im Vakuumtrockenschrank bei 35°C und 300 mbar getrocknet.
- Ausbeute:: 72.5 g
- ¹H-NMR:: δ = 1.4 (t, 3 H), 4.2 (q, 2 H), 4.6 (NH₂, 2 H), 7.0-7.7 (Ar, 4 H), 9.1 (CONH, 1 H)
- MS:: 361 (2M+H, 15), 181 (M+H, 100)
- HPLC:: 96 F1%

### Beispiel B: Herstellung von Buttersäureethylimidat-Hydrochlorid

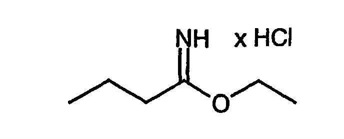

Buttersäurenitril (85 ml, 839 mmol) wird in absolutem Ethanol (65 ml, 961 mmol) gelöst und auf 0°C bis 5°C abgekühlt. Unter weiterer Kühlung leitet man Chlorwasserstoffgas (ca. 40 g, ca. 1,1 mol, Differenzwägung der Druckflasche) in die Lösung ein. Die Reaktionsmischung wird 96 Stunden bei ca. 4°C belassen, dann unter Rühren mit 260 ml Diisopropylether versetzt, auf -20°C abgekühlt und bei 0°C über eine Fritte abgesaugt. Die Kristalle werden zügig (das Produkt ist hygroskopisch!) zweimal mit je 50 ml kaltem Diisopropylether gewaschen, und luftdicht verschlossen im Kühlschrank gelagert.
- Ausbeute:: 131,6 g
- ¹H-NMR:: δ = 0.9 (t, 3 H), 1.3 (t, 3 H), 1.6 (m, 2 H), 2.6 (t, 2 H), 4.5 (q, 2 H), 11.3+12.2 (NH, 1 H)
- MS:: 115 (M+, 10), 10 (20), 87 (22), 72 (25), 70 (22), 59 (50), 43 (100), 41 (40), 36 (22)

### Beispiel C: Herstellung von 2-Ethoxybenzoesäure-N'-(1-iminobutyl)-hydrazid-Hydrochlorid

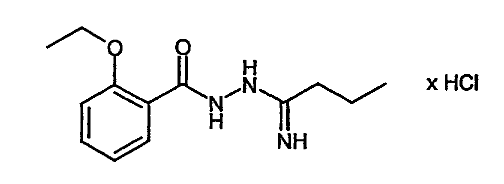

Buttersäureethylimidat-Hydrochlorid aus Bsp. B (71,5 g, 472 mmol) wird mit Triethylamin (65 ml, 472 mmol) vorgelegt und auf 0°C abgekühlt. 2-Ethoxybenzoesäurehydrazid aus Bsp. A (85 g, 472 mmol) wird in 250 ml iso-Propanol suspendiert und zu der vorgelegten Mischung des Buttersäureethylimidats gegeben. Die Reaktionsmischung wird 48 Stunden bei Raumtemperatur gerührt, der Feststoff (Triethylamin-Hydrochlorid) wird abgetrennt, und in die Mutterlauge wird langsam bei 0°C bis 5°C 25 %ige Salzsäure (150 ml, 1,18 mol) eingetropft. Die Kristallsuspension wird 1 Stunde bei 0°C bis 5°C nachgerührt, das Kristallisat wird über eine Fritte abgesaugt, mit 100 ml kaltem iso-Propanol gewaschen und über Nacht im Vakuumtrockenschrank bei 30°C und 300 mbar getrocknet.
- Ausbeute:: 96.4 g
- ¹H-NMR:: δ = 1.0 (t, 3 H), 1.4 (t, 3 H), 1.8 (m, 2 H), 2.6 (t, 2 H), 4.2 (q, 2 H), 7.0-7.9 (Ar, 4 H), 9.0+10.0+11.8 (NH, 2 H), 10.4 (CONH, 1 H)
- MS:: 499 (2M+H, 20), 250 (M+H, 100)
- HPLC:: 86 F1%

### Beispiel D: Herstellung von 2-Ethoxy-N-(4-methyl-2-propyl-imidazol-1-yl)-benzamid

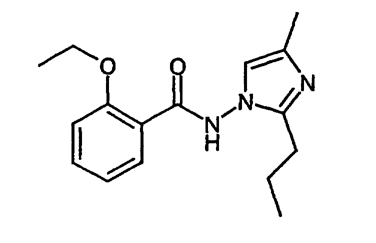

### Amin-Variante:

2-Ethoxybenzoesäure-N'-(1-iminobutyl)-hydrazid aus Bsp. C (5 g, 17,5 mmol) wird in 100 ml ges. Natriumhydrogencarbonatlösung aufgenommen und anschließend mit zweimal 100 ml Dichlormethan extrahiert. Die Phasen werden getrennt, und die organische Phase wird getrocknet, vollständig eingeengt und in 300 ml Acetonitril aufgenommen. In einem separaten Kolben werden Chloraceton (3,0 ml, 35 mmol), Triethylamin (5,5 ml, 17,5 mmol) und Kaliumiodid (2,9 g, 17,5 mmol) in 15 ml Acetonitril vorgelegt und auf 50°C erwärmt. Zu der vorgelegten Mischung wird bei 50°C innerhalb von ca. 1 Stunde die oben hergestellte Lösung von 2-Ethoxybenzoesäure-N'-(1-iminobutyl)-hydrazid in Acetonitril zugegeben, und die Mischung wird 4 Stunden bei 50°C gehalten. Die entstandene Suspension wird auf Raumtemperatur abgekühlt, der Feststoff wird abgetrennt, das Filtrat vollständig eingeengt und in 200 ml Dichlormethan aufgenommen. Die Dichlormethanlösung wird zweimal mit je 200 ml ges. Natriumchloridlösung gewaschen. Die organische Phase wird mit Natriumsulfat getrocknet und am Rotationsverdampfer eingeengt.
Ausbeute: 3.7 g
Die spektroskopischen Daten waren identisch mit dem nach der Carbonat-Variante hergestellten Produkt.

### Carbonat Variante:

2-Ethoxybenzoesäure-N'-(1-iminobutyl)-hydrazid aus Bsp. C (200 g, 700 mmol), Kaliumcarbonat (193,5 g, 1400 mmol) und Kaliumiodid (23,2 g, 140 mmol) werden in 1400 ml Aceton bei 15°C vorgelegt und bis zum vollständigen Umsatz (ca. 20 Stunden) bei 15°C nachgerührt. Es werden 1400 ml Wasser zugegeben, und das Aceton wird im Vakuum abdestilliert. Die Suspension wird abgekühlt, das Kristallisat wird isoliert und gewaschen. Das so erhaltene Kristallisat wird in 600 ml Xylol (Isomerengemisch) re-suspendiert, und Wasser wird durch Destillation im Vakuum bei 85 bis 115°C entfernt. Die erhaltene Lösung wird abgekühlt und das Produkt durch Animpfen und Zugabe von Cyclohexan kristallisiert. Das Kristallisat wird isoliert, mit Cyclohexan gewaschen und im Vakuum getrocknet.
- Ausbeute:: 144.9 g
- ¹H-NMR:: δ = 0.9 (t, 3 H), 1.4 (t, 3 H), 1.6 (m, 2 H), 2.1 (s, 3 H), 2.5 (t, 2 H), 4.2 (q, 2 H), 6.8 (1H, Imidazol), 7.0-7.6 (Ar, 4 H), 11.1 (CONH, 1 H)
- MS:: 575 (2M+H, 15), 451 (10), 288 (M+H, 100)
- HPLC:: 94 F1%

### Herstellungsbeispiele

### Beispiel 1: 2-(2-Ethoxyphenyl)-5-methyl-7-propyl-3H-imidazo[5,1-f]-triazin-4-on (via Iodierungsroute)

### 1a) 2-Ethoxy-N-(5-iod-4-methyl-2-propyl-imidazol-1-yl)-benzamid

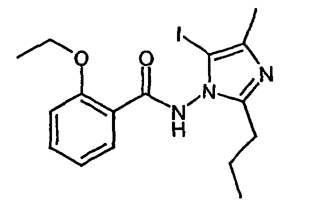

2-Ethoxy-N-(4-methyl-2-propyl-imidazol-1-yl)-benzamid aus Bsp. D (3,5 g, 12,2 mmol) wird in 480 ml einer Dioxan/Wasser-Mischung gelöst und mit Natriumcarbonat (3,9 g, 36,6 mmol) und Iod (6,8 g, 26,8 mmol) versetzt. Der Ansatz wird unter Lichtausschluss 24 Stunden bei Raumtemperatur gerührt. Die Reaktionsmischung wird mit 50 ml Essigester versetzt und zweimal mit je 50 ml gesättigter Natriumsulfitlösung gewaschen. Die vereinigten wässrigen Phasen werden zweimal mit je 50 ml Essigester reextrahiert, und die vereinigte organische Phase wird mit Natriumsulfat getrocknet und einrotiert.
- Ausbeute:: 4.1 g
- ¹H-NMR:: δ = 0.9 (t, 3 H), 1.4 (t, 3 H), 1.6 (m, 2 H), 2.1 (s, 3 H), 2.6 (t, 2 H), 4.2 (q, 2 H), 7.1-7.6 (Ar, 4 H), 11.1 (CONH, 1 H)
- MS:: 414 (M+H, 100), 222 (10), 149 (30), 121 (65)
- HPLC:: 82 F1%

### 1b) N-(5-Cyano-4-methyl-2-propyl-imidazol-1-yl)-2-ethoxy-benzamid

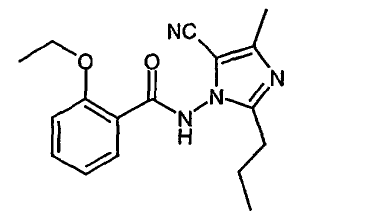

2-Ethoxy-N-(5-iod-4-methyl-2-propyl-imidazol-1-yl)-benzamid aus Bsp. 1a (0,5 g, 1,2 mmol) und Kupfer(I)cyanid (0,7 g, 7,5 mmol) werden in 10 ml Pyridin vorgelegt und drei Stunden auf 100°C erwärmt. Die Reaktionsmischung wird auf Raumtemperatur abgekühlt, mit 50 ml Dichlormethan versetzt und durch Filtration vom Feststoff befreit. Das Filtrat wird dreimal mit je 50 ml Wasser gewaschen, und die organische Phase wird mit 50 ml Dichlormethan reextrahiert. Die vereinigte organische Phase wird mit Natriumsulfat getrocknet und eingeengt. Der Rückstand wird zur Entfernung von restlichem Pyridin in 50 ml Dichlormethan aufgenommen und sechsmal mit je 100 ml Wasser gewaschen. Die organische Phase wird mit Natriumsulfat getrocknet und einrotiert. Abschließend wird der Rückstand mit 100 ml Toluol versetzt und eingeengt.
- Ausbeute:: 0.3 g
- ¹H-NMR:: δ = 0.9 (t, 3 H), 1.4 (t, 3 H), 1.7 (m, 2 H), 2.3 (s, 3 H), 2.6 (t, 2 H), 4.2 (q, 2 H), 7.1-7.7 (Ar, 4 H), 11.5 (CONH, 1 H)
- MS:: 313 (M+H, 100), 149 (25), 149 (30), 120 (15)
- HPLC:: 82 F1%

### 1c) 2-(2-Ethoxyphenyl)-5-methyl-7-propyl-3H-imidazo[5,1-f]-triazin-4-on

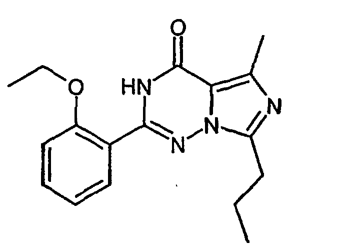

48,5 %ige Schwefelsäure (5 ml) wird auf 0°C abgekühlt, und N-(5-Cyano-4-methyl-2-propyl-imidazol-1-yl)-2-ethoxy-benzamid aus Bsp. 1b (0,2 g, 0,6 mmol) wird zugesetzt. Die Suspension wird 2 Stunden bei Raumtemperatur und dann 1 Stunde bei 70°C gerührt. Der Ansatz wird mit 30 ml Wasser verdünnt und mit dreimal je 30 ml Dichlormethan extrahiert. Die vereinigte organische Phase wird mit Natriumsulfat getrocknet und eingeengt.
- Ausbeute:: 0.1 g
- ¹H-NMR:: δ = 1.0 (t, 3 H), 1.6 (t, 3 H), 1.9 (m, 2 H), 2.8 (s, 3 H), 3.3 (t, 2 H), 4.3 (q, 2 H), 7.0-8.2 (Ar, 4 H), 10.3 (CONH, 1 H)
- MS:: 313 (M+H, 100), 149 (25),151 (40), 121 (15)
- HPLC:: 80 F1%

### Beispiel 2: 2-(2-Ethoxyphenyl)-5-methyl-7-propyl-3H-imidazo[5,1-f]-triazin-4-on (via Bromierungsroute)

### 2a) N-(5-Brom-4-methyl-2-propyl-imidazol-1-yl)-2-ethoxy-benzamid

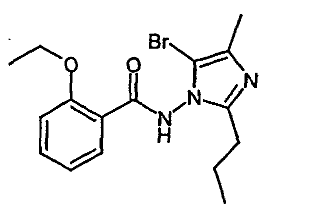

2-Ethoxy-N-(4-methyl-2-propyl-imidazol-1-yl)-benzamid aus Bsp. D (50 g, 174 mmol) wird in Essigsäure vorgelegt. Tropfenweise wird Brom (12,5 ml, 243,6 mmol) gelöst in Essigsäure bei Raumtemperatur zugegeben. Der Ansatz wird bis zum vollständigen Umsatz (bis zu 3 Stunden) bei Raumtemperatur nachgerührt. Zur Aufarbeitung wird der Ansatz mit Wasser und Ethanol (oder optional Aceton) versetzt. Das Produkt wird durch Neutralstellen mit Natronlauge ausgefällt. Das Kristallisat wird isoliert, gewaschen und im Vakuum getrocknet.
- Ausbeute:: 59.7 g
- ¹H-NMR:: δ = 0.9 (t, 3 H), 1.4 (t, 3 H), 1.65 (m, 2 H), 2.1 (s, 3 H), 2.55 (t, 2 H), 4.2 (q, 2 H), 7.1-7.7 (Ar, 4 H), 11.2 (CONH, 1 H)
- MS:: 366 (M+H, 100), 203 (30), 149 (20),121 (30)
- HPLC:: 99 F1%

### 2b) N-(5-Cyano-4-methyl-2-propyl-imidazol-1-yl)-2-ethoxy-benzamid

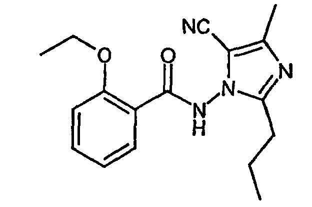

N-(5-Brom-4-methyl-2-propyl-imidazol-1-yl)-2-ethoxy-benzamid aus Bsp. 2a (0,5 g, 1,2 mmol) und Kupfer(I)cyanid (0,3 g, 3,5 mmol) werden in 12,5 ml Pyridin vorgelegt und 6 Stunden auf 100°C erwärmt. Zu der Mischung wird Kaliumiodid (23 mg, 0,56 mmol) gegeben, und weitere 6 Stunden bei 100°C nachgerührt. Die Reaktionsmischung wird auf Raumtemperatur abgekühlt, mit 50 ml Ethylacetat versetzt und mit verdünnter alkalischer H₂O₂-Lösung gewaschen. Die wässerige Phase wird mit Ethylacetat reextrahiert, die vereinigte Ethylacetat-Phase wird eingeengt, abschließend mit Toluol azeotropiert und erneut eingeengt.
- Ausbeute:: 0.3 g
- ¹H-NMR:: δ = 0.9 (t, 3 H), 1.4 (t, 3 H), 1.7 (m, 2 H), 2.3 (s, 3 H), 2.6 (t, 2 H), 4.2 (q, 2 H), 7.1-7.7 (Ar, 4 H), 11.5 (CONH, 1 H)
- MS:: 313 (M+H, 100), 149 (25), 149 (30), 120 (15)
- HPLC:: 82 F1%

### 2c) 2-(2-Ethoxyphenyl)-5-methyl-7-propyl-3H-imidazo[5,1-f]-triazin-4-on

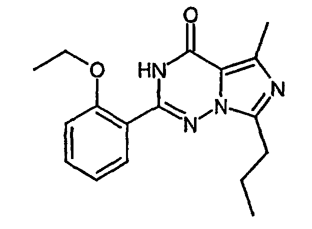

48,5 %ige Schwefelsäure (5 ml) wird auf 0°C abgekühlt, und N-(5-Cyano-4-methyl-2-propyl-imidazol-1-yl)-2-ethoxy-benzamid aus Bsp. 2b (0,2 g, 0,6 mmol) wird zugesetzt. Die Suspension wird 2 Stunden bei Raumtemperatur und dann 1 Stunde bei 70°C gerührt. Der Ansatz wird mit 30 ml Wasser verdünnt und mit dreimal je 30 ml Dichlormethan extrahiert. Die vereinigte organische Phase wird mit Natriumsulfat getrocknet und eingeengt.
- Ausbeute:: 0.1 g
- ¹H-NMR:: δ = 1.0 (t, 3 H), 1.6 (t, 3 H), 1.9 (m, 2 H), 2.8 (s, 3 H), 3.3 (t, 2 H), 4.3 (q, 2 H), 7.0-8.2 (Ar, 4 H), 10.3 (CONH, 1 H)
- MS:: 313 (M+H, 100), 149 (25),151 (40), 121 (15)
- HPLC:: 80 F1%

## Patentansprüche

1. Verfahren zur Herstellung von Verbindungen der Formel (I) 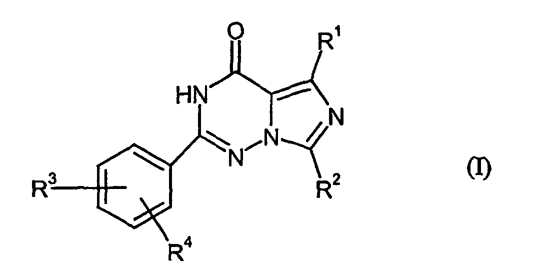 worin
R¹ für Wasserstoff oder geradkettiges oder verzweigtes Alkyl mit bis zu 4 Kohlenstoffatomen steht,
R² für geradkettiges Alkyl mit bis zu 4 Kohlenstoffatomen steht,
R³ und R⁴ gleich oder verschieden sind und für Wasserstoff, geradkettiges oder verzweigtes Alkyl mit bis zu 6 Kohlenstoffatomen, Hydroxy oder für geradkettiges oder verzweigtes Alkoxy mit bis zu 6 Kohlenstoffatomen stehen,
umfassend die Umsetzung von Verbindungen der Formel (II) 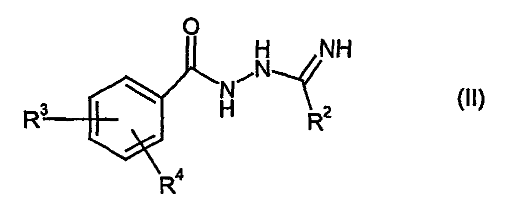 worin R², R³ und R⁴ die vorstehend angegebene Bedeutung haben,
mit Verbindungen der Formel (III) 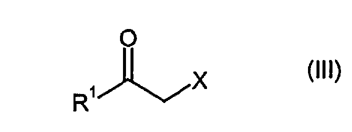 worin
R¹ die vorstehend angegebene Bedeutung hat,
X für Halogen steht;
in Gegenwart einer Base und gegebenenfalls eines Metalliodids in einem organischen Lösungsmittel zu Verbindungen der Formel (IV) 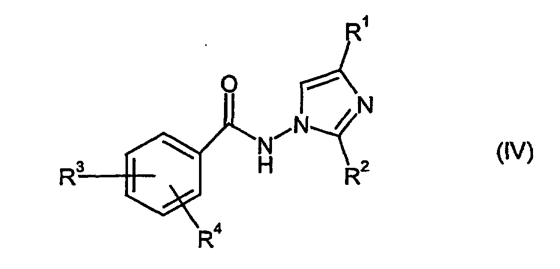 worin R¹, R², R³ und R⁴ die vorstehend angegebene Bedeutung haben,
und die anschließende Umsetzung
[A] mit Iod in Gegenwart einer Base in einem Lösungsmittel, anschließende Reaktion mit einem Metallcyanid in einem Lösungsmittel und Umsetzung mit einer Säure
oder
[B] mit Brom im sauren Medium, anschließende Reaktion mit einem Metallcyanid in einem Lösungsmittel und Umsetzung mit einer Säure.

2. Verfahren nach Anspruch 1, **dadurch gekennzeichnet, dass**
R¹ geradkettiges Alkyl mit bis zu 4 Kohlenstoffatomen,
R² geradkettiges Alkyl mit bis zu 4 Kohlenstoffatomen,
R³ und R⁴ gleich oder verschieden voneinander Wasserstoff oder geradkettiges oder verzweigtes Alkoxy mit bis zu 4 Kohlenstoffatomen
bedeuten.

3. Verfahren nach Anspruch 1, **dadurch gekennzeichnet, dass**
R¹ Methyl oder Ethyl,
R² n-Propyl,
R³ Wasserstoff
R⁴ Ethoxy
bedeuten.

4. Verfahren nach einem der vorhergehenden Ansprüche, **dadurch gekennzeichnet, dass** in der Verbindung der Formel (III) X für Chlor steht.

5. Verfahren nach einem der vorhergehenden Ansprüche, **dadurch gekennzeichnet, dass** die Umsetzung der Verbindungen der Formel (II) und (III) in Gegenwart von Kaliumiodid und Triethylamin durchgeführt wird.

6. Verfahren nach einem der vorhergehenden Ansprüche, **dadurch gekennzeichnet, dass** die Umsetzung der Verbindungen der Formel (II) und (III) in Gegenwart von Kaliumcarbonat durchgeführt wird.

7. Verfahren nach einem der vorhergehenden Ansprüche, **dadurch gekennzeichnet, dass** die Umsetzung der Verbindungen der Formel (IV) mit Iod unter Lichtausschluss in Gegenwart von Natriumcarbonat und anschließend mit CuCN in Pyridin durchgeführt wird.

8. Verfahren nach einem der vorhergehenden Ansprüche, **dadurch gekennzeichnet, dass** die Umsetzung der Verbindungen der Formel (IV) mit Brom in Gegenwart von Essigsäure und anschließend mit CuCN in Pyridin in Gegenwart von Kaliumiodid durchgeführt wird.

9. Verfahren nach einem der vorhergehenden Ansprüche, **dadurch gekennzeichnet, dass** im abschließenden Schritt als Säure Schwefelsäure verwendet wird.

10. Verfahren nach einem der vorhergehenden Ansprüche, **dadurch gekennzeichnet, dass** die Verbindungen der Formel (II) durch Umsetzung von Verbindungen der Formel (V) 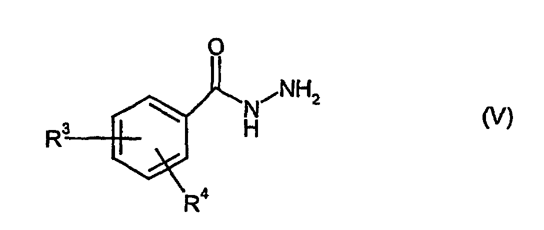 worin
R³ und R⁴ die in Anspruch 1 angegebene Bedeutung haben,
mit Verbindungen der Formel (VI) 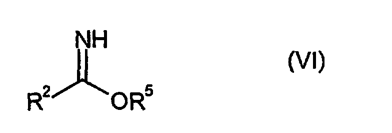 worin
R² die in Anspruch 1 angegebene Bedeutung hat
R⁵ für C₁₋₆-Alkyl steht,
in einem organischen Lösungsmittel in Gegenwart einer Base hergestellt werden.

## Claims

1. Process for preparing compounds of the formula (I) 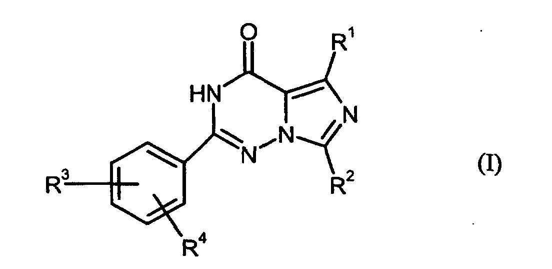 in which
R¹ represents hydrogen or straight-chain or branched alkyl having up to 4 carbon atoms,
R² represents straight-chain alkyl having up to 4 carbon atoms,
R³ and R⁴ are identical or different and represent hydrogen, straight-chain or branched alkyl having up to 6 carbon atoms, hydroxyl or represent straight-chain or branched alkoxy having up to 6 carbon atoms,
comprising reaction of compounds of the formula (II) 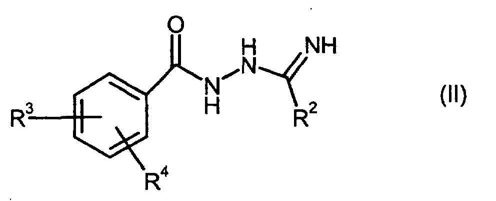 in which R², R³ and R⁴ have the meaning indicated above,
with compounds of the formula (III) 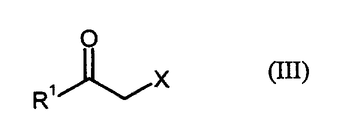 in which
R¹ has the meaning indicated above,
X represents halogen;
in the presence of a base and, where appropriate, of a metal iodide in an organic solvent to give compounds of the formula (IV) 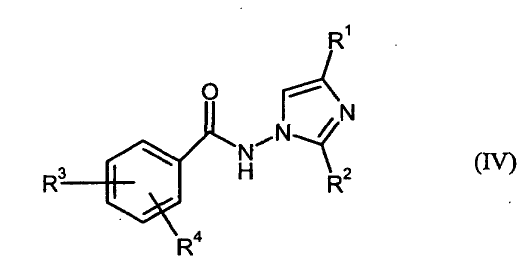 in which R¹, R², R³ and R⁴ have the meaning indicated above,
and subsequent reaction
[A] with iodine in the presence of a base in a solvent, subsequent reaction with a metal cyanide in a solvent and reaction with an acid
or
[B] with bromine in acidic medium, subsequent reaction with a metal cyanide in a solvent and reaction with an acid.

2. Process according to Claim 1, **characterized in that**
R¹ denotes straight-chain alkyl having up to 4 carbon atoms,
R² denotes straight-chain alkyl having up to 4 carbon atoms,
R³ and R⁴ denote, identically or differently from one another, hydrogen or straight-chain or branched alkoxy having up to 4 carbon atoms.

3. Process according to Claim 1, **characterized in that**
R¹ denotes methyl or ethyl,
R² denotes n-propyl,
R³ denotes hydrogen
R⁴ denotes ethoxy.

4. Process according to any of the preceding claims, **characterized in that** X in the compound of the formula (III) represents chlorine.

5. Process according to any of the preceding claims, **characterized in that** the reaction of the compounds of the formula (II) and (III) is carried out in the presence of potassium iodide and triethylamine.

6. Process according to any of the preceding claims, **characterized in that** the reaction of the compounds of the formula (II) and (III) is carried out in the presence of potassium carbonate.

7. Process according to any of the preceding claims, **characterized in that** the reaction of the compounds of the formula (IV) with iodine is carried out with the exclusion of light in the presence of sodium carbonate and subsequently with CuCN in pyridine.

8. Process according to any of the preceding claims, **characterized in that** the reaction of the compounds of the formula (IV) with bromine is carried out in the presence of acetic acid and subsequently with CuCN in pyridine in the presence of potassium iodide.

9. Process according to any of the preceding claims, **characterized in that** sulphuric acid is used as acid in the concluding step.

10. Process according to any of the preceding claims, **characterized in that** the compounds of the formula (II) are prepared by reacting compounds of the formula (V) 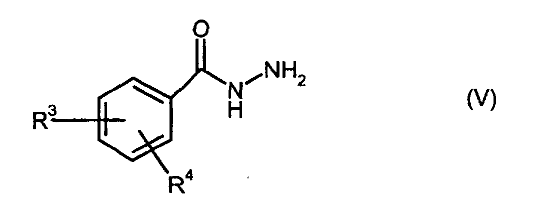 in which
R³ and R⁴ have the meaning indicated in Claim 1,
with compounds of the formula (VI) 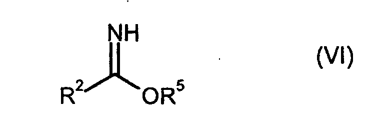 in which
R² has the meaning indicated in Claim 1,
R⁵ represents C₁₋₆-alkyl,
in an organic solvent in the presence of a base.

## Revendications

1. Procédé de production de composés de formule (I) 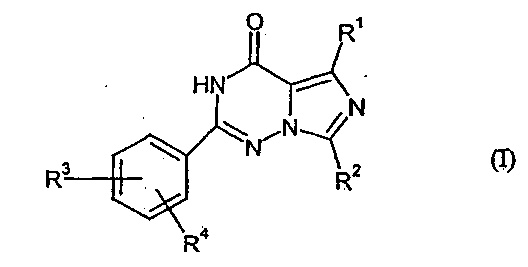 dans laquelle
R¹ représente l'hydrogène ou un reste alkyle linéaire ou ramifié ayant jusqu'à 4 atomes de carbone,
R² est un reste alkyle linéaire ayant jusqu'à 4 atomes de carbone,
R³ et R⁴ sont identiques ou différents et représentent l'hydrogène, un reste alkyle linéaire ou ramifié ayant jusqu'à 6 atomes de carbone, un groupe hydroxy ou un reste alkoxy linéaire ou ramifié ayant jusqu'à 6 atomes de carbone,
comprenant la réaction de composés de formule (II) 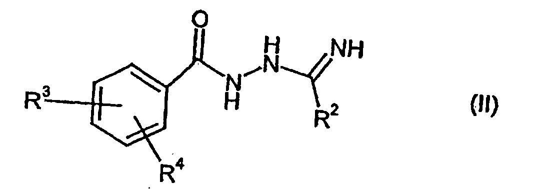 dans laquelle R², R³ et R⁴ ont la définition indiquée ci-dessus,
avec des composés de formule (III) 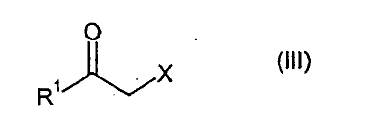 dans laquelle
R¹ a la définition indiquée ci-dessus,
X est un halogène ;
en présence d'une base et, le cas échéant, d'un iodure métallique, dans un solvant organique, pour obtenir des composés de formule (IV) 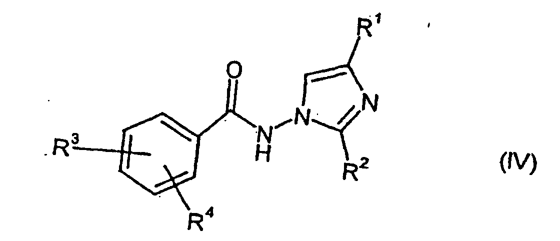 dans laquelle R¹, R², R³ et R⁴ ont la définition indiquée ci-dessus,
et la réaction subséquente
[A] avec l'iode en présence d'une base dans un solvant, suivie de la réaction avec un cyanure métallique dans un solvant et de la réaction avec un acide
ou bien
[B] avec le brome en milieu acide, suivie de la réaction avec un cyanure métallique dans un solvant et de la réaction avec un acide.

2. Procédé suivant la revendication 1, **caractérisé en ce que**
R¹ est un reste alkyle linéaire ayant jusqu'à 4 atomes de carbone,
R² est un reste alkyle linéaire ayant jusqu'à 4 atomes de carbone,
R³ et R⁴, identiques ou différents l'un de l'autre, représentent l'hydrogène ou un reste alkoxy linéaire
ou ramifié ayant jusqu'à 4 atomes de carbone.

3. Procédé suivant la revendication 1, **caractérisé en ce que**
R¹ est un reste méthyle ou éthyle,
R² est un reste n-propyle,
R³ est l'hydrogène
R⁴ est un reste éthoxy.

4. Procédé suivant l'une des revendications précédentes, **caractérisé en ce que** X représente le chlore dans le composé de formule (III).

5. Procédé suivant l'une des revendications précédentes, **caractérisé en ce que** la réaction des composés de formules (II) et (III) est conduite en présence d'iodure de potassium et de triéthylamine.

6. Procédé suivant l'une des revendications précédentes, **caractérisé en ce que** la réaction des composés de formules (II) et (III) est conduite en présence de carbonate de potassium.

7. Procédé suivant l'une des revendications précédentes, **caractérisé en ce que** la réaction des composés de formule (IV) est conduite avec l'iode à l'abri de la lumière en présence de carbonate de sodium puis avec CuCN dans la pyridine.

8. Procédé suivant l'une des revendications précédentes, **caractérisé en ce que** la réaction des composés de formule (IV) est conduite avec le brome en présence d'acide acétique puis avec CuCN dans la pyridine en présence d'iodure de potassium.

9. Procédé suivant l'une des revendications précédentes, **caractérisé en ce que** l'acide utilisé dans l'étape finale est l'acide sulfurique.

10. Procédé suivant l'une des revendications précédentes, **caractérisé en ce que** les composés de formule (II) sont préparés par réaction des composés de formule (V) 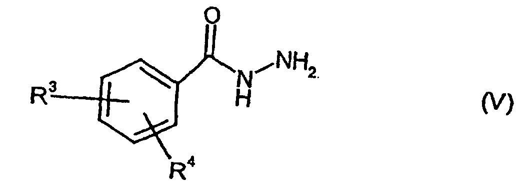 dans laquelle
R³ et R⁴ ont la définition indiquée dans la revendication 1,
avec des composés de formule (VI) 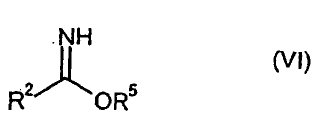 dans laquelle
R² a la définition indiquée dans la revendication 1
R⁵ est un reste alkyle en C₁ à C₆,
dans un solvant organique, en présence d'une base.
